# EUROPEAN PATENT SPECIFICATION

(11) **EP 0 323 605 B1**
(45) Date of publication and mention of the grant of the patent: **26.01.1994**
(21) Application number: 88121314.4
(22) Date of filing: 20.12.1988
(51) Int. Cl.: G01N 33/558, G01N 33/543

(54) **Chromatographic binding assay devices and methods**
Chromatographische Bindungstesteinrichtungen und Verfahren
Appareil et méthodes de test chromatographique de liaison

(30) Priority: 21.12.1987 US 135810; 14.12.1988 US 282978
(43) Date of publication of application: 12.07.1989
(73) Proprietor: ABBOTT LABORATORIES, Abbott Park, Illinois 60064-3500 (US)
(72) Inventor: Devereaux, Sharon Murphy, Gurnee, IL 60031 (US); Wilcox, James L., Lake Bluff, IL 60044 (US); Holzman, Thomas Fredric, Libertyville, IL 60048 (US); Gordon, Julian, Lake Bluff, IL 60044 (US); Ching, Shanfun, Libertyville, IL 60048 (US)
(74) Representative: Modiano, Guido, Dr.-Ing.

(56) References cited:
- EP-A- 0 157 071
- EP-A- 0 271 204
- WO-A-86/03839
- DE-C- 3 445 816
- FR-A- 2 356 944
- FR-A- 2 514 511

## Description

### BACKGROUND OF THE INVENTION

### 1 . Field of the Invention

The present invention relates to a novel test device for detecting an analyte in a test sample by means of a chromatographic binding assay. In particular, this invention relates to a novel test device for the chromatographic quantitation or semiquantitation of analytes and to methods for using such devices.

### 2. Description of Related Art

In the development of the medical diagnostics field, there has been explosive growth in the number of substances to be detected in physiological test samples. Various analytical procedures are commonly used in diagnostic assays to determine the presence and/or amount of these substances of interest or clinical significance. These clinically significant or interesting substances are commonly referred to as analytes. Diagnostic assays have become an indispensable means for detecting analytes in test samples, and for the most part the medical profession has used highly automated clinical laboratories and sophisticated equipment for these determinations.

There is, however, an expanding need for having analytical capabilities in doctors' offices and in the home. Together with the diagnosis of disease, there is a growing need to monitor the effects of drug therapy and chronic illness, to detect the use of drugs of abuse and to detect the presence of contaminants.

Numerous approaches have been developed toward this end, depending to varying degrees on instrumental or visual observation of the assay result. Typical of these methods are the so called "dipstick" and "flow-through" devices and methods. The dipstick generally uses a plastic strip with a reagent-containing matrix layered thereon. A test sample is applied to the device, and the presence of the analyte is indicated by a visually detectable signal such as a color-forming reaction. The flow-through device generally uses a porous material with a reagent-containing matrix layered thereon or incorporated therein. Test sample is applied to and flows through the porous material, and analyte in the sample reacts with the reagent(s) to produce a detectable signal on the porous material.

While such devices have proven useful for the qualitative determination of the presence of analytes they are not particularly useful for making quantitative distinctions. In addition, typical conventional assay devices often require a number of manipulative steps, for example, the addition and incubation of assay reagents and the need for intermediate washing steps. These complex devices and methods are time consuming and require the close attention of a user who, depending on the device, must time the various assay steps and in some cases measure the reagents to be added. Accordingly, there remains a need for new devices which are easy to use, accurate and rapid and which allow for quantitative or semi-quantitative analytical testing.

### 3 . Brief Description of the Relevant Literature

Hochstrasser (U.S. Pat. 4,059,407) discloses a dipstick device which can be immersed in a biological fluid to semi-quantitate analyte in the fluid. Semi-quantitation of the analyte is accomplished by using a series of reagent-containing pads wherein each pad in the series will produce a detectable color (i.e., a positive result) in the presence of an increasing amount of analyte. Also of interest in the area of dipstick devices are U.S. Pat. Nos. 3,802,842, 3,915,639 and 4,689,309.

Deutsch et a. describe a quantitative chromatographic test strip device in U.S. Pat. Nos. 4,094,647, 4,235,601 and 4,361,537. The device comprises a material capable of transporting a solution by capillary action, i.e., wicking. Different areas or zones in the strip contain the reagents needed to produce a detectable signal as the analyte is transported to or through such zones. The device is suited for both chemical assays and binding assays which are typified by the binding reaction between an antigen and its complementary antibody.

Many variations on the Deutsch et al. device have been disclosed. For example, Tom et al. (U.S. Pat. No. 4,366,241) disclose a bibulous strip with an immunosorbing zone to which the test sample is applied. Grubb et al. (U.S. Pat. No. 4,168,146) describe the use of a porous test strip material to which is covalently bound an antigen-specific antibody. In performance of an assay, the test strip is immersed in a solution suspected of containing an antigen, and capillary migration of the solution up the test strip is allowed to occur. As the antigen moves up the test strip it binds to the immobilized antigen-specific antibody. The presence of antigen is then determined by wetting the strip with a second antigen-specific antibody to which a fluorescent or enzyme label is covalently bound. Quantitative testing can be achieved by measuring the length of the strip that contains bound antigen. Variations on such a test strip are disclosed in U.S. Pat. Nos. 4,435,504 which employs a two enzyme indicator system; 4,594,327 which discloses the addition of a binding agent to whole blood samples which causes the red blood cells to aggregate at the area of the strip adjacent to the air/liquid interface; and 4,757,004 which discloses a means for controlling the shape of the fluid front migrating along the test strip. Zuk et al., Enzyme Immunochromatography - A Quantitative Immunoassay Requiring No Instrumentation, *Clinical Chemistry*, 31(7): 1144-1150, 1985, further describe the assay principle. Also of interest are U.S. Pat. Nos. 4,298,688; 4,517,288 and 4,740,468; E.P. Publication Nos. 88,636; 259,157; and 267,006; and DE Pat. No. 3,445,816 which describes a sheet-like diagnostic device comprising one or several strips, arranged behind one another, having zones situated one behind another, wherein each zone is readily accessible from above and below for the addition of reagents and a quantitative determination of an analyte.

Flow-through assay devices are described in U.S. Pat. Nos. 3,825,410; 3,888,629; 4,446,232; 4,587,102; 4,632,901; 4,637,978 and 4,727,019; and E.P. Publication Nos. 212,603; 217,403 and 249,851.

### SUMMARY OF THE INVENTION

The present invention provides assay devices, methods and kits for determining the presence or amount of an analyte in a test sample containing or suspected of containing an analyte of interest. The assay device comprises a chromatographic material having a proximal end and a distal end, wherein the test sample can travel from the proximal end to the distal end by capillary action. The chromatographic material contains a capture reagent, immobilized in a capture situs, that is capable of binding to a member selected from the group consisting of the analyte, an ancillary specific binding member and an indicator reagent. An application pad is in fluid flow contact with the proximal end of the chromatographic material; the application pad receives the test sample and contains a diffusive indicator reagent capable of migrating from the application pad to the chromatographic material. The indicator reagent is capable of binding to a member selected from the group consisting of the analyte, an ancillary specific binding member and the capture reagent. The binding of the indicator reagent results in a detectable signal at the capture situs thereby indicating the presence or amount of the analyte in the test sample. The capture reagent can be positioned upon or within the chromatographic material in a variety of configurations.

The assay method involves placing a test sample suspected of containing the analyte onto the application pad, thereby contacting the indicator reagent. The test sample and the indicator reagent are transferred to the chromatographic material, and migrate through the chromatographic material to contact the capture reagent, resulting in the production of a detectable signal at the capture situs.

The test kit of the invention includes the analytical device; preferably the device includes the reagents used in the assay. The device and test kit can optionally and preferably include a housing which prevents the test sample from directly contacting the chromatographic material and which includes means for applying the test sample to the application pad.

### BRIEF DESCRIPTION OF THE FIGURES

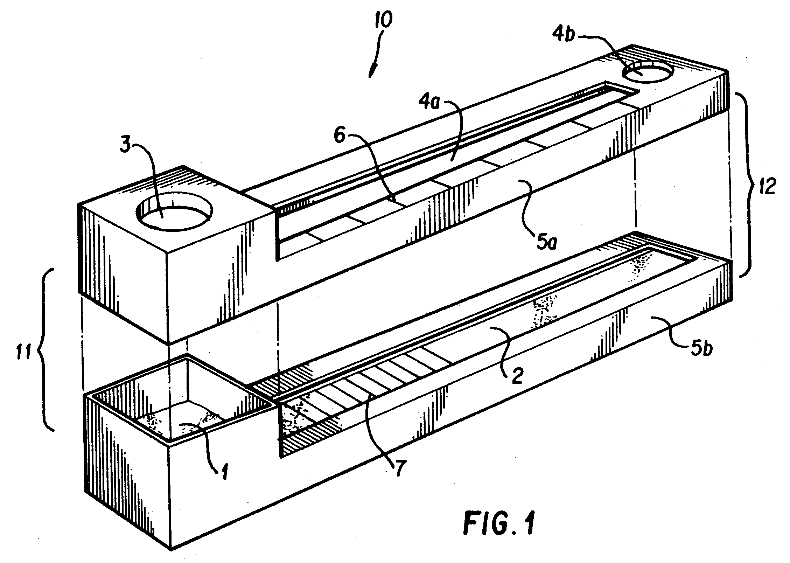

Figure 1 is an exploded view of a device of the present invention having optional, preferred embodiments.

### DETAILED DESCRIPTION OF THE INVENTION

This invention provides assay devices and methods, where the devices use a chromatographic material capable of transporting liquids and an application pad which functions to regulate the flow of test samples to the chromatographic material, to filter the test samples and to mix the assay reagents. The assay reagents are incorporated within the application pad and the chromatographic material and display a detectable signal in the presence of analyte. By varying the configuration of reagent-containing sites on the chromatographic material, qualitative and quantitative displays of assay results can be obtained. The devices can be formatted to perform binding assays, such as the complementary binding of an antigen and antibody. The detectable signal resulting from the binding assay can then be detected by instrumentation or direct visual observation.

In use of an assay device according to the invention, a test sample, which can be a liquid which is used directly as obtained from the source, or which has been pretreated in a variety of ways so as to modify its character, is introduced to the device through the application pad. The sample passes through the application pad, where it contacts one or more reagents involved in producing the assay reaction, to the chromatographic material through which the test sample will wick and in which it will encounter one or more additional reagents involved in producing the detectable signal.

Virtually any liquid sample can be used, so long as the sample has a reasonable rate of passage through the application pad and a reasonable rate of transport along the chromatographic material. The test sample can be derived from any desired source, such as a physiological fluid, for example, blood, saliva, ocular lens fluid, cerebral spinal fluid, sweat, urine, milk, ascites fluid, mucous, synovial fluid, peritoneal fluid, amniotic fluid or the like. The fluid can be pretreated prior to use, such as preparing plasma from blood, diluting viscous fluids, or the like; methods of treatment can also involve separation, filtration, distillation, concentration, inactivation of interfering components, and the addition of reagents. Besides physiological fluids, other liquid samples such as water, food products and the like can be used. In addition, a solid can be used once it is modified to form a liquid medium.

The present invention is particularly advantageous in that it combines several elements to form a novel assay device with which a one-step assay can be performed. The novel device simplifies the assay protocols by decreasing the number of manual steps required for its use, thereby reducing the risk of errors during use. The combination of elements in the present invention also enables the use of predetermined amounts of reagents incorporated within the device, thereby avoiding the need for reagent measurements and additions by the user. Furthermore, the reagents are situated in the device in such a way as to make the assay substantially self-performing and to facilitate the detection and quantitation of the assay results.

### I. DEFINITIONS

A "specific binding member", as used herein, is a member of a specific binding pair, i.e., two different molecules wherein one of the molecules through chemical or physical means specifically binds to the second molecule. In addition to antigen and antibody specific binding pairs, other specific binding pairs include, as examples without limitation, biotin and avidin, carbohydrates and lectins, complementary nucleotide sequences such as the probe and capture nucleic acids used in hybridization reactions with a target nucleic acid sequence as the analyte, complementary peptide sequences, effector and receptor molecules, enzyme cofactors and enzymes, enzyme inhibitors and enzymes, a peptide sequence and an antibody specific for the sequence or the entire protein, and the like. Furthermore, specific binding pairs can include members that are analogs of the original specific binding member, for example an analyte-analog. If the specific binding member is an immunoreactant it can be, for example, an antibody, antigen, hapten, or complex thereof, and if an antibody is used, it can be a monoclonal or polyclonal antibody, a recombinant protein or antibody, a mixture(s) or fragment(s) thereof, as well as a mixture of an antibody and other specific binding members. The details of the preparation of such antibodies and their suitability for use as specific binding members are well known to those skilled in the art.

When an immunoreactive specific binding member is attached to the chromatographic material of the present invention, the device is referred to as an "immunochromatograph", and the corresponding method of analysis is referred to as "immunochromatography". Immunochromatography, as used herein, encompasses both sandwich and competitive immunoassay techniques.

An "analyte", as used herein, is the compound or composition to be detected or measured in the test sample. In a binding assay, the analyte will have at least one epitope or binding site for which there exists a naturally occurring, complementary specific binding member or for which a specific binding member can be prepared. "Analyte", also includes any antigenic substances, haptens, antibodies, and combinations thereof. The analyte of interest in an assay can be, for example, a protein, a peptide, an amino acid, a nucleic acid, a hormone, a steroid, a vitamin, a pathogenic microorganism for which polyclonal and/or monoclonal antibodies can be produced, a natural or synthetic chemical substance, a contaminant, a drug including those administered for therapeutic purposes as well as those administered for illicit purposes, and metabolites of or antibodies to any of the above substances.

Illustrative of the so-called "drugs of abuse" which are suitable as analytes for this invention are the following: codeine, morphine, heroin, cocaine (and its metabolite benzoylecgonine), methamphetamine, phencyclidine, phenothiazines, tetrahydrocannabinol and the like. Illustrative of the therapeutic and/or prophylactic pharmaceutical agents, which are suitable for detection or measurement by the present invention, are theophylline, digoxin, digitoxin, gentamicin, tobramycin, amikacin, kanamycin, netilmicin, streptomycin, phenobarbital, dilantin, procainamide, N-acetylprocainamide, lidocaine. quinidine, propranolol, ethosuximide, acetaminophen, acetylsalicylic acid, carbamazepineprimidone, valproic acid, methotrexate, dibekacin-chloramphenicol, vancomycin, disopyramide, amitriptyline, desipramine, imipramine, nortriptyline and the like.

Examples of the hormones which are suitable as analytes for this invention are the following: thyroid stimulating hormone (TSH), human chorionic gonadotropin (hCG), luteinizing hormone (LH) and follicle stimulating hormone (FSH). An especially preferred hormone analyte in pregnancy testing is hCG.

Pathogenic microorganisms suitable for analysis by the present invention include those microorganisms disclosed in U.S. Patent No. 4,366,241. Illustrative of some of these microorganisms are those associated with urinary tract infections, such as *Streptococcus pyogenes, Streptococcus salivarus, Escherichia coli, Staphylococcus aureus, Klebsiella pneumonia, Proteus mirabilis* and the like. The microorganisms, when assayed by the present invention, may be intact, lysed, ground or otherwise fragmented and the resulting composition or portion thereof assayed. Preferably, the microorganisms are assayed intact.

The term "analyte-analog", as used herein, refers to a substance which cross-reacts with an analyte-specific binding member, although it may do so to a greater or a lesser extent than does the analyte itself. The analyte-analog can include a modified analyte as well as a fragmented or synthetic portion of the analyte molecule so long as the analyte-analog has at least one epitopic site in common with the analyte of interest.

"Label", as used herein, is any substance which is attached to a specific binding member and which is capable of producing a signal that is detectable by visual or instrumental means. Various suitable labels for use in the present invention can include chromogens; catalysts; fluorescent compounds; chemiluminescent compounds; radioactive labels; direct visual labels including colloidal metallic and non-metallic particles, dye particles, enzymes or substrates, or organic polymer latex particles; liposomes or other vesicles containing signal producing substances; and the like.

A large number of enzymes suitable for use as labels are disclosed in U.S. Patent No. 4,275,149, columns 19-23. A particularly preferred enzyme/substrate signal producing system useful in the present invention is the enzyme alkaline phosphatase wherein the substrate used is nitro blue tetrazolium-5-bromo-4-chloro-3-indolyl phosphate or a derivative or analog thereof.

In an alternative signal producing system, the label can be a fluorescent compound where no enzymatic manipulation of the label is required to produce a detectable signal. Fluorescent molecules such as fluorescein, phycobiliprotein, rhodamine and their derivatives and analogs are suitable for use as labels in this reaction.

In an especially preferred embodiment, a visually detectable, colored particle can be used as the label component of the indicator reagent, thereby providing for a direct colored readout of the presence or concentration of the analyte in the sample without the need for further signal producing reagents. Materials for use as the colored particles are colloidal metals, such as gold, and dye particles as disclosed in U.S. Pat. Nos. 4,313,734 and 4,373,932. Non-metallic colloids, such as colloidal selenium particles and organic polymer latex particles, can also be used as labels .

A "signal producing component", as used herein, refers to any substance capable of reacting with another assay reagent or the analyte to produce a reaction product or signal that indicates the presence of the analyte and that is detectable by visual or instrumental means. "Signal production system", as used herein, refers to the group of assay reagents that are needed to produce the desired reaction product or signal. For example, one or more signal producing components can be used to react with a label and generate the detectable signal, i.e., when the label is an enzyme, amplification of the detectable signal is obtained by reacting the enzyme with one or more substrates or additional enzymes to produce a detectable reaction product.

An "ancillary specific binding member", as used herein, refers to any member of a specific binding pair which is used in the assay in addition to the specific binding members of the capture reagent and the indicator reagent and which becomes a part of the final binding complex. One or more ancillary specific binding members can be used in an assay. For example, an ancillary specific binding member can be capable of binding the analyte, as well as a second specific binding member to which the analyte itself could not attach.

### II. REAGENTS AND MATERIALS

### a. Binding Assay Reagents

In the present invention, binding assays involve the specific binding of the analyte and/or an indicator reagent (comprising a label attached to a specific binding member) to a capture reagent (comprising a second specific binding member) which immobilizes the analyte and/or indicator reagent on a chromatographic material or which at least slows the migration of the analyte or indicator reagent through the chromatographic material.

The label, as described above, enables the indicator reagent to produce a detectable signal that is related to the amount of analyte in the test sample. The specific binding member component of the indicator reagent enables the indirect binding of the label to the analyte, to an ancillary specific binding member or to the capture reagent. The selection of a particular label is not critical, but the label will be capable of generating a detectable signal either by itself, such as a visually detectable signal generated by colored organic polymer latex particles, or in conjunction with one or more additional signal producing components, such as an enzyme/substrate signal producing system. A variety of different indicator reagents can be formed by varying either the label or the specific binding member; it will be appreciated by one skilled in the art that the choice involves consideration of the analyte to be detected and the desired means of detection.

The capture reagent, in a binding assay, is used to facilitate the observation of the detectable signal by substantially separating the analyte and/or the indicator reagent from other assay reagents and the remaining components of the test sample. The capture reagent of the present invention is a specific binding member, such as those described above. In a binding assay, the capture reagent is immobilized on the chromatographic material to form a "capture situs", i.e., that region of the chromatographic material having one or more capture reagents non-diffusively attached thereto.

### b. Application pad

The application pad is in fluid flow contact with one end of the chromatographic material, referred to as the proximal end, such that the test sample can pass or migrate from the application pad to the chromatographic material; fluid flow contact can include physical contact of the application pad to the chromatographic material as well as the separation of the pad from the chromatographic strip by an intervening space or additional material which still allows fluid flow between the pad and the strip. Substantially all of the application pad can overlap the chromatographic material to enable the test sample to pass through substantially any part of the application pad to the proximal end of the strip of chromatographic material. Alternatively, only a portion of the application pad might be in fluid flow contact with the chromatographic material. The application pad can be any material which can transfer the test sample to the chromatographic material and which can absorb a volume of test sample that is equal to or greater than the total volume capacity of the chromatographic material.

Materials preferred for use in the application pad include nitrocellulose, porous polyethylene frit or pads and glass fiber filter paper. The material must also be chosen for its compatibility with the analyte and assay reagents, for example, glass fiber filter paper was found to be the preferred application pad material for use in a human chorionic gonadotropin (hCG) assay device.

In addition, the application pad contains one or more assay reagents either diffusively or non-diffusively attached thereto. Reagents which can be contained in the application pad include, but are not limited to, indicator reagents, ancillary specific binding members, and any signal producing system components needed to produce a detectable signal. For example, in a binding assay it is preferred that an indicator reagent be diffusively attached to the application pad; this eliminates the need to combine test sample and indicator reagent prior to use in an assay. The isolation of assay reagents in the application pad also keeps interactive reagents separate and facilitates the manufacturing process.

The application pad receives the test sample, and the wetting of the application pad by the sample will perform at least two functions. First, it will dissolve or reconstitute a predetermined amount of reagent contained by the pad. Secondly, it will initiate the transfer of both the test sample and the freshly dissolved reagent to the chromatographic material. In some instances, the application pad serves a third function as both an initial mixing site and a reaction site for the test sample and reagent.

In one preferred embodiment of the present invention, gelatin is used to encompass all or part of the application pad. Typically, such encapsulation is produced by overcoating the application pad with fish gelatin. The effect of this overcoating is to increase the stability of the reagent contained by the application pad. The application of test sample to the overcoated application pad causes the gelatin to dissolve and thereby enables the dissolution of the reagent. In an alternative embodiment of the present invention, the reagent containing application pad is dried or lyophilized to increase the shelf-life of the device. Lyophilized application pads were found to produce stronger signals than air dried application pads, and the lyophilized application pads maintained stability for longer periods. The reagents contained in the application pad are rehydrated with the addition of test sample to the pad.

In another preferred embodiment, the present invention can be further modified by the addition of a filtration means. The filtration means can be a separate material placed above the application pad or between the application pad and the chromatographic material, or the material of the application pad itself can be chosen for its filtration capabilities. The filtration means can include any filter or trapping device used to remove particles above a certain size from the test sample. For example, the filter means can be used to remove red blood cells from a sample of whole blood, such that plasma is the fluid received by the application pad and transferred to the chromatographic material. Such filter means are disclosed by U.S. Pat. No. 4,477,575 and WO Application No. 86/02192, published April 23, 1987.

A still further preferred modification of the present invention involves the use of an additional layer or layers of porous material placed between the application pad and the chromatographic material or overlaying the application pad. Such an additional pad or layer can serve as a means to control the rate of flow of the test sample from the application pad to the chromatographic material. Such flow regulation is preferred when an extended incubation period is desired for the reaction of the test sample and the reagent(s) in the application pad. Alternatively, such a layer can contain an additional assay reagent(s) which is preferably isolated from the application pad reagents until the test sample is added, or it can serve to prevent unreacted assay reagents from passing to the chromatographic material.

When small quantities of non-aqueous or viscous test samples are applied to the application pad, it may be necessary to employ a wicking solution, preferably a buffered wicking solution, to carry the reagent(s) and test sample from the application pad and through the the chromatographic material. When an aqueous test sample is used, a wicking solution generally is not necessary but can be used to improve flow characteristics or adjust the pH of the test sample. In immunochromatography, the wicking solution typically has a pH range from about 5.5 to about 10.5, and more preferably from about 6.5 to about 9.5. The pH is selected to maintain a significant level of binding affinity between the specific binding members in a binding assay. When the label component of the indicator reagent is an enzyme, however, the pH also must be selected to maintain significant enzyme activity for color development in enzymatic signal production systems. Illustrative buffers include phosphate, carbonate, barbital, diethylamine, tris, 2-amino-2-methyl-l-propanol and the like. The wicking solution and the test sample can be combined prior to contacting the application pad or they can be contacted to the application pad sequentially.

### c. Chromatographic Material

The chromatographic material of the assay device of the present invention can be any suitably absorbant, porous or capillary possessing material through which a solution containing the analyte can be transported by a wicking action. Natural, synthetic, or naturally occurring materials that are synthetically modified, can be used as the chromatographic material including, but not limited to: cellulose materials such as paper, cellulose, and cellulose derivatives such as cellulose acetate and nitrocellulose; fiberglass; cloth, both naturally occurring (e.g., cotton) and synthetic (e.g., nylon); porous gels such as silica gel, agarose, dextran, and gelatin; porous fibrous matrixes; starch based materials, such as Sephadex^{®} brand cross-linked dextran chains; ceramic materials; films of polyvinyl chloride and combinations of polyvinyl chloride-silica; and the like. The chromatographic material should not interfere with the production of a detectable signal. The chromatographic material should have a reasonable inherent strength, or strength can be provided by means of a supplemental support.

A preferred chromatographic material is nitrocellulose. When nitrocellulose is used, however, the material of the application pad should be chosen for its ability to premix the test sample and the first reagent, i.e., fluid-flow through a nitrocellulose membrane is laminar and does not provide the more turbulent flow characteristics which allows the initial mixing of test sample and application pad reagents within the chromatographic material. If nitrocellulose is used as the chromatographic material, then Porex^{®} hydrophilic polyethylene frit or glass fiber filter paper are appropriate application pad materials because they enable the mixing and reaction of the test sample and application pad reagents within the application pad and before transfer to the chromatographic material. An especially preferred chromatographic material is glass fiber filter paper.

The particular dimensions of the chromatographic material will be a matter of convenience, depending upon the size of the test sample involved, the assay protocol, the means for detecting and measuring the signal, and the like. For example, the dimensions may be chosen to regulate the rate of fluid migration as well as the amount of test sample to be imbibed by the chromatographic material.

As discussed above, in a binding assay the capture situs can be formed by directly or indirectly attaching the capture reagent to the chromatographic material. Direct attachment methods include adsorption, absorption and covalent binding such as by use of (i) a cyanogen halide, e.g., cyanogen bromide or (ii) by use of glutaraldehyde. It is preferred, however, to retain or immobilize the desired reagent on the chromatographic material indirectly through the use of insoluble microparticles to which the reagent has been attached. The means of attaching a reagent to the microparticles encompasses both covalent and non-covalent means, that is adhered, absorbed or adsorbed. It is preferred that capture reagents be attached to the microparticles by covalent means. By "retained and immobilized" is meant that the particles, once on the chromatographic material, are not capable of substantial movement to positions elsewhere within the material. The particles can be selected by one skilled in the art from any suitable type of particulate material composed of polystyrene, polymethylacrylate, polyacrylamide, polypropylene, latex, polytetrafluoroethylene, polyacrylonitrile, polycarbonate, glass or similar materials. The size of the particles is not critical, although generally it is preferred that the average diameter of the particles be smaller than the average pore or capillary size of the chromatographic material.

It is also within the scope of this invention to attach more than one reagent to the microparticles to be immobilized within the chromatographic material. For example, to slow or prevent the diffusion of the detectable reaction product in an enzyme/substrate signal producing system, the substrate can be immobilized within the chromatographic material. The substrate can be immobilized by direct attachment to the chromatographic material by methods well known in the art, but it is preferred that the substrate also be immobilized by being covalently bound to insoluble microparticles which have been deposited in and/or on the chromatographic material.

The size of the particles may vary depending upon the type of chromatographic material used as well as the type of material from which the particle is made. For example, in a glass fiber chromatographic material, glass and polystyrene particles should be of sufficient size to become entrapped or immobilized in the pores of the chromatographic material and not move when confronted by the migrating fluid. In the same glass fiber matrix, much smaller latex particles can be used because the latex particles unexpectedly affix themselves to the glass fibers by an unknown mechanism; see EP-A-217 403.

Thus, unlike the pore size dependent glass and plastic particles, the latex particles are pore sized independent so that lot-to-lot variations in pore size of the chromatographic material will not adversely affect the performance of the device. As a result, one particularly preferred binding assay device uses latex particles, having capture reagent attached thereto, distributed in a glass fiber chromatographic material. The distribution of the microparticles or other reagents onto or into the matrix of the chromatographic material can be accomplished by reagent printing techniques as disclosed in EP-A-268 237.

The capture reagent, signal producing component or reagent-coated microparticles can be deposited singly or in various combinations on or in the chromatographic material in a variety of configurations to produce different detection or measurement formats. For example, a reagent can be deposited as a discrete situs having an area substantially smaller than that of the entire chromatographic material.

Alternatively, the reagent can be distributed over the entire chromatographic material in a substantially uniform manner to form a capture situs or reaction situs that substantially includes the entire chromatographic material. The extent of signal production along the length of the capture situs or reaction situs, or the distance of the detectable signal from the proximal end of the chromatographic material, is then related to the amount of analyte in the test sample. The amount of analyte can be determined by the comparison of the length or distance of the resulting signal to those observed for calibrated standards of analyte.

In another embodiment, the reagent can be distributed as a narrow stripe running from about the proximal to about the distal end of the chromatographic material. Use of the narrow stripe, rather than a uniform distribution of reagent, can serve to sharpen the image of the detectable signal on the chromatographic material. Furthermore, more than one narrow parallel stripe can be distributed along the length of the chromatographic material, wherein the reagent within each stripe is directed to a different analyte, thereby forming a multi-analyte assay device. As an addition to the configurations in which the length or distance of analyte travel is measured, a scale of appropriate symbols, numbers or letters can be imprinted upon the chromatographic material to aid in the measurement and thus the quantification of analyte.

In another embodiment, the reagent can be distributed more lightly at one end of the chromatographic material than at the other. In a competitive binding assay, this deposition of capture reagent in a gradient fashion provides for greater sensitivity at the end of the chromatographic material having the lighter distribution, because of the more rapid displacement of the indicator reagent from the capture reagent binding sites by the analyte.

In alternative embodiments, the appropriate assay reagents can be distributed in any pattern convenient for detection including, but not limited to, numerals, letters, dots and symbols such as "+/-", "%" or the like which display the detectable signal upon completion of the assay.

In yet another embodiment, the reagents can be distributed as a series of parallel bars which traverse the width of the chromatographic material and which are spaced from about the proximal end of the chromatographic material to about the distal end, thereby creating a ladder-like capture situs configuration. As with the narrow-stripe configuration, the bars and the intervening spaces serve to sharpen the image of the signal produced on the chromatographic material. The number of bars at which signal is detectable can be counted and correlated to the amount of analyte in the test sample. When the bars are spaced closely together, the device provides less analytical sensitivity but greater amounts of analyte can be measured. Alternatively, by spacing the bars further apart, increasingly greater sensitivity can be obtained. It is also within the scope of this invention to vary the sensitivity within different portions of the chromatographic material depending upon whether greater discrimination sensitivity for the analyte is required at the high end or low end of its concentration range. Another variation of the parallel bar configuration involves the use of multiple capture or reaction reagents wherein the reagents within the capture and detection sites are directed to a different analyte, thereby forming a multi-analyte assay device.

In the multiple bar chromatographic assays using antibodies as specific binding members and visually detectable colloidal particles, the visual readout of the amount of analyte present is a function of the competitive displacement of the colloid-labeled antibody/analyte complex from the capture reagent antibody by analyte which is not bound to the colloid-labeled antibody. This can result in proximal bars which contain analyte bound to the capture reagent but which contain no detectable indicator reagent. Due to this competitive displacement, the antibodies used in the assay should be chosen based upon the desirable range of analyte concentrations to be detected or measured as well as the affinity of those antibodies for the analyte, where affinity is defined as the reciprocal of the apparent dissociation constant (Kd, having physical dimensions of moles/liter). If the first specific binding member is an antibody coated upon the colloidal particle label, then it is believed that the antibody should have a dissociation constant of less than the lowest analyte concentration to be detected, i.e., the antibody on the colloidal particle should bind at least fifty percent of available analyte at equilibrium. For example, the Kd can be less than the lowest analyte concentration by an amount of from about two to 1000 fold. More preferably, the Kd will be such that the colloid-labeled antibody will bind about 90% to about 99% of the analyte present. Most preferably, the Kd will be such that the colloid-labeled antibody will bind about 99.9% of the analyte. In addition, the analyte to be detected is preferably present in the test sample in an amount equal to or greater than the total amount of the colloid-labeled antibody used in the assay. While it is likely that the system will function to some extent even if the labeled-antibody Kd is within a factor of two higher or lower than the low end of the assay range, as the Kd is further reduced the assay performance will improve. Specifically, the assay will exhibit a lower background of unbound indicator reagent on the chromatographic strip, and complete indicator reagent capture will occur in a shorter distance on the strip.

The dissociation constant of the capture reagent antibody should have the same set of preferable values as does the labeled antibody.

The parallel bar capture situs or reaction situs can be further modified such that the bar nearest the application pad is larger than the following bars. The use of such a configuration in a competitive binding assay enables the capture of substantially all of the indicator reagent in the first bar when there is no analyte in the test sample. This configuration requires that there be sufficient capture reagent in the first bar to immobilize substantially all of the indicator reagent used in the assay. Alternatively, the first bar or reaction site can contain a sufficient amount of reagent to inhibit a binding reaction unless a threshold amount of analyte is present in the sample.

A variation of the parallel bar configuration involves the detection of more than one signal to provide assay controls. For example in a binding assay, at least one of the proximal bars can contain microparticles having analyte-specific binding members affixed thereto whereas the most distal bar(s) contain microparticles having a label-specific binding member affixed thereto. When a sufficient amount of indicator reagent is used, the distal bar(s) serve as a built-in control to display that the assay has been completed, i.e., the indicator reagent will become attached to the distal bars whatever the assay result and thereby indicate both that the test sample and assay reagents have passed through the proximal bars and that the indicator reagent is active. A variety of display formats or patterns as described above can incorporate assay controls to confirm the efficacy of the assay reagents or device, the completion of the assay or the proper sequence of assay steps.

It is also within the scope of this invention to have a reagent, at the distal end of the chromatographic material, which indicates the completion of a binding assay (i.e., end of assay indicator) by changing color upon contact with the test solution, wicking solution or a signal producing component. Reagents which would change color upon contact with a test solution containing water are the dehydrated transition metal salts, such as CuSO₄, Co(NO₃)₂, and the like. The pH indicator dyes can also be selected to respond to the pH of the buffered wicking solution. For example, phenolphthalein changes from clear to intense pink upon contact with a wicking solution having a pH range between 8.0-10.0.

Reagents can be added directly to either the application pad or the chromatographic material during the performance of the assay. The preferred embodiment of the invention, however, involves the incorporation of all necessary assay reagents into the assay device so that only a liquid test sample need be contacted to the application pad to perform the assay. Therefore, one or more assay reagents can be present in either or both the application pad or chromatographic material of the present invention.

The present invention further provides kits for carrying out binding assays. For example, a kit according to the present invention can comprise the assay device with its incorporated reagents as well as a wicking solution and/or test sample pretreatment reagents as described above. Other assay components known to those skilled in the art, such as buffers, stabilizers, detergents, bacteria inhibiting agents and the like can also be present in the assay device and wicking solution.

### III. Housing

The present invention optionally includes a nonreactive enclosure around the device. Preferably, the housing encloses at least the application pad to facilitate receiving and/or containing a certain volume of the test sample and/or wicking solution (Figure 1).

The device of the present invention may be partially or completely encased in the non-reactive enclosure. While the entire chromatographic material need not be encased, it is preferred that a sufficient portion of the application pad should be encased to prevent applied test sample from contacting the chromatographic material without first passing through the application pad.

Depending upon the particular assay protocols involved and the methods of device construction, the housing will be removable or irremovable, will provide for one or more windows, and will normally be of a sturdy, inert and impermeable material. The housing will provide mechanical protection as well as support for the application pad and chromatographic material but will not interfere with the performance of the assay. The housing can be made of an opaque or a transparent material. The size of the window can be varied such that a housing made of opaque material can partially or completely hide certain portions of the chromatographic material.

In **Figure 1**, an assay device **10** is shown with an upper **5a** and lower **5b** non-reactive enclosure and having a proximal end **11** and a distal end **12**. The enclosures **5a** and **5b** enclose the application pad **1** and the chromatographic material **2** as described above. The application pad **1** is located at the proximal end **11** of the chromatographic material. The upper enclosure **5a** contains at least one opening or transparent window **4a**. Window **4a** permits the observation of the chromatographic material **2** and the signal produced thereon by the signal producing system. An optional window **4b** at the distal end **12** of upper enclosure **5a** permits observation of a color produced when the test solution or other assay reagent contacts the optional assay completion zone. An application port **3** enables the application of test sample and wicking solution to the application pad **1**. As an additional option, the upper enclosure **5a** may contain calibration marks **6** to facilitate quantitation of the analyte in the test sample. The capture situs **7** represents an alternative embodiment, as described above, wherein reagents are distributed as a series of parallel bars which traverse the width of the chromatographic material.

### IV. DIAGNOSTIC ASSAYS

In a competitive binding assay, the liquid front of the test sample, or of the test solution comprising test sample and optional wicking solution, contains both the indicator reagent, e.g., labeled analyte, from the application pad and the unlabeled analyte from the test sample. Accordingly, both the labeled and unlabeled analyte are carried from the application pad to the chromatographic material where they are transported distally by the advancing liquid front. During their distal migration, the labeled and unlabeled analyte compete for binding sites on capture reagent, e.g., antibody, immobilized on the chromatographic material.

Because the labeled analyte and unlabeled analyte compete for binding sites on the capture reagent, the labeled analyte migrates equidistantly with the unlabeled analyte. Thus, the greater the concentration of analyte in the sample, the greater the distal migration of the labeled and unlabeled analyte through the chromatographic material. Analogously, the lower the concentration of analyte in the sample, the shorter the distal migration of the labeled analyte. The distance of distal migration of the indicator reagent, and thus the concentration of the analyte in the test sample, are then determined by the detectable signal produced by the label of the indicator reagent.

In a sandwich binding assay , the migrating test solution or test sample contains both the dissolved indicator reagent from the application pad and the analyte from the test sample. Accordingly, both the indicator reagent and analyte are carried distally by the advancing liquid front. Moreover, during their migration, the indicator reagent can bind to the analyte to form an indicator reagent/analyte complex. As the wicking liquid transports the indicator reagent/analyte complex through the chromatographic material, the immobilized capture reagent also binds to the analyte to render the indicator reagent/analyte complex immobilized. Thus, the indicator reagent/analyte complex is able to advance only so far as capture reagent binding sites on the chromatographic material are already occupied and no longer available for further binding. Consequently, the greater the concentration of analyte in the test sample, the further the distal migration of the indicator reagent/analyte complex through the chromatographic material before immobilization occurs. Analogously, the lower the concentration of analyte in the test sample, the shorter the distal migration. Accordingly, in a sandwich assay, the concentration of analyte in the test sample is directly proportional to the distance of distal migration by the indicator reagent/analyte complex.

### EXAMPLES

### Example 1

### Immunochromatographic Assay for hCG

### a) Indicator reagent preparation - colloidal gold/mouse anti-hCG antibody conjugate

A gold reference solution (20 ml, Fisher SO-G-91, 1.0 ml = 1.0 mg of gold from gold chloride in distilled water) was mixed with distilled water (180 ml) and heated until boiling. One percent sodium citrate (5 ml, in distilled water) was added to the solution and swirled while heating. The solution underwent a color change from yellow to colorless (45 seconds) to dark reddish-purple (two minutes). The solution was removed from the heat when red first appeared. The solution was cooled, filtered and stored at 4° C until used.

Mouse anti-hCG antibodies (approximately 500 µg, 10 µg/ml) were added to the colloidal gold solution (50 ml) and 1% Potassium carbonate (575 µl, to adjust pH to 8). After mixing for one minute, 2% fish gelatin (250 µl; Inotec, Wohlen, Switzerland) was added to quench the reaction. The solution was mixed for another five minutes and was then centrifuged at 2990 x g at 4 °C for 25 minutes (DuPont Sorvall SS34 rotor). The resulting pellet was collected, and the remaining supernatant was centrifuged at 8630 x g at 4 °C for 25 minutes. The second pellet was collected, and both pellets were combined to yield one milliliter of colloidal gold/mouse anti-hCG antibody conjugate to which was added 10% polyethyleneglycol (PEG, 10 µl, MW 20,000) and 10% sodium azide (10 µl).

### b) Anti-hCG antibody-coated particles

To 345 microliters of latex microparticles (10% solids [v/v] in water; 0.21 microns in diameter), in a one dram vial, were added sequentially 100 nM 2-[N-morpholino]ethanesulfonic acid buffer (MES; 132 µl), 1-ethyl-3-(3-dimethylaminopropyl)-carbodiimide (1-EDAC; 55µl at 5 mg/ml), and affinity purified polyclonal anti-hCG antibody (2.1233 ml at 4.7 mg/ml). The mixture was incubated for about three hours. Thereafter, the particles were washed three times by centrifuging and replacing the supernatant with 0.1% polyoxyethylenesorbitan ester (Tween-20) in water.

After the last wash, the particles were passed four times through a 25 gauge needle and then diluted to a 0.69% solids final concentration with 0.01 M phosphate buffer, pH 7.4. The resulting final anti-hCG antibody concentration was approximately 2.0 mg/ml.

### c) Chromatographic material preparation

The capture situs was prepared by reagent printing the anti-hCG antibody-coated latex particles of Example 1.b (concentrated to 1.30% solids) onto a 100 mm x 70 mm glass fiber filter (Gelman A/E, Gelman Sciences Incorporated, Ann Arbor, MI). The reagent printing was performed substantially in accordance with the printing techniques disclosed in co-owned and copending U.S. Patent Application Serial No. 931,476, filed November, 17 1986 using a 51 micron diameter orifice (at 12 psi) and passing the material under the orifice at a rate of four inches per second, with reagent dispensed at 31.3 mg/sec. The capture situs was printed as thirty-three, one millimeter wide, horizontal bars. An overcoat of 1% fish gelatin in water was applied, and the chromatographic material was allowed to dry. The filter was cut perpendicular to the horizontal bars to produce a set of 6 mm x 70 mm strips having thirty-three bars traversing the width of the strip. Two microliters of colloidal gold/mouse anti-hCG antibody indicator reagent (prepared substantially in accordance with the method described in Example 1.a) was then applied to the proximal end of each strip and air dried.

### d) Assay protocol

To a point (e.g., origin) at the bottom of the individual strips was added 100 microliters of one of the following reference solutions:
1. 0 mlU hCG in urine standard,
2. 250 mlU hCG in urine standard,
3. 500 mlU hCG in urine standard, and
4. high positive urine specimen from pregnant woman (mlU hCG concentration not determined).

The strips were placed in 500 microliters of 10% alkaline treated casein in 0.05 M Tris buffer (pH 7.2), and the test sample was allowed to migrate to the distal end of the strip. Excess conjugate was then rinsed from the strip with distilled water. The number of bars containing immobilized indicator reagent were recorded, as was the migration distance from the origin. The results are presented in Table 1.

**Table 1**

| Assay for hCG Using a Colloidal Gold Conjugate | | | |
|---|---|---|---|
| Strip No. | mlU hCG | No. of Visualized Bars | Distance From Origin |
| 1 | 0 | None | - |
| 2 | 250 | 1 | 18 mm |
| 3 | 500 | 7 | 29 mm |
| 4 | high positive | 33 | 58 mm |

The data of Table 1 demonstrated that as the analyte concentration increased the number of bars which displayed signal also increased, and the signal was displayed further along the chromatographic material.

### Example 2

### Immunochromatographic Assay For E. coli

### a. Indicator reagent preparation - colloidal selenium/anti-E. coli antibody conjugate

Using silanized glassware, SeO₂ (750 mg) was dissolved in boiled water (25 L) which was then added to boiling water (25 L) with vigorous stirring. Approximately one minute later, sodium ascorbate (56.5 ml; 1% w/v in boiled water) was added to the boiling solution and caused a reddish suspension to form. The solution was boiled for another ten minutes and then allowed to cool to room temperature, whereupon it was centrifuged for 30 minutes at approximately 5,000 x g. The reddish brown precipitate was separated from the aqueous phase, washed in water and recentrifuged twice more. The precipitate was separated into black (denser) granular material, which was undesired, and into a red-brown finer material, which was desired. The reddish-brown material was resuspended in a sufficient quantity of Tris buffered saline to make approximately 200 milliliters of selenium suspension (TBS; containing 50 mM Tris-HCl, pH 7.4, 0.9% NaCl and 0.05% w/v sodium ascorbate)

The pH of the selenium suspension was adjusted to pH 7.5 with 0.01 N K₂CO₃ (about 2.5 ml), and to 10 ml of that suspension was added approximately 100 µg of bovine anti-whole *E. coli* antibody dissolved in 0.9% NaCl (20.71 µl). The mixture was gently agitated for about two minutes. Solid bovine serum albumin (BSA; 100 mg) was added to the mixture, and the mixture was agitated until the BSA dissolved. The mixture was centrifuged for 15 minutes at 4 °C and 50,000 x g. The resulting colloidal selenium/anti-E. coli antibody indicator reagent was resuspended and recentrifuged two times in TBS with 1% BSA and finally resuspended in that same solution to a final concentration of about 10 µg/ml.

### b) Anti-whole E. coli antibody-coated particles

To 2.5 milliliters of polystyrene latex microparticles (2% solids [v/v] in 0.1 M MES, pH 5.0) were added sequentially EDAC (1.0 ml in MES 500 µMoles), bovine anti-whole *E. coli* antibody (0.5 ml, 1.0 mg/ml) and MES (1.0 ml). The mixture was incubated at room temperature, while stirring, for about two hours. Thereafter, the particles were centrifuged in phosphate buffered saline (PBS, 5.0 ml) and washed two times by centrifuging and replacing the supernatant with TBS/BSA. After the last wash, the particles were resuspended in TBS (0.1M Tris HCl, pH 7.4 and 0.9% NaCl, 1% BSA and 0.1% NaN₃).

### c) Chromatographic material preparation

The capture situs was prepared by reagent printing the bovine anti-whole *E. coli* antibody-coated polystyrene latex microparticles (prepared substantially in accordance with the method described in Example 2.b and printed substantially in accordance with the method described in Example 1.c) onto a 100 mm x 50 mm glass fiber filter (Gelman A/E). The capture reagent was printed as a series of horizontal bars approximately 0.08 inches apart. An overcoat of 1% fish gelatin (Inotec, Wohlen, Switzerland) in water was applied, and the chromatographic material was allowed to dry. The chromatographic material was then cut into 3 mm x 50 mm strips such that the cut was perpendicular to the horizontal bars.

### d) Assay protocol

A test sample of *E. coli*, suspended at 10⁸ per milliliter of 0.9% NaCl, was mixed 1:1 (v/v) with the indicator reagent of Example 2.a to form a test solution. The test solution was briefly incubated at room temperature whereby the indicator reagent began to complex with the *E. coli* of the test sample. The proximal end of a strip of Example 2.c was submerged in the test solution. The test solution migrated through the strip, and the accumulation of indicator reagent/analyte complex immobilized on the strip was detectable by the appearance of brown-red bars indicating the formation of a "sandwich" immunocomplex at the capture situs.

### Example 3

### One Step Immunochromatographic Assay For Benzoylecgonine (a Cocaine Metabolite)

### a. Indicator reagent preparation - selenium colloid/anti-benzoylecgonine antibody conjugate

A 3% stock solution of SeO₂ (50 ml, 3 g/ml, Aldrich) was diluted in water (5 L) and pH adjusted first with 2% NaOH to 4.4 -4.5 and then with 2% K₂CO₃ to 4.95 -5.05. The solution was heated to 75 -80 °C. The solution was then mixed with a 5% ascorbate solution (75 ml), and this reaction mixture was heated in a 70 °C water bath while stirring for five minutes. The solution was then mixed with 5% ascorbate (6 ml/L), and this reaction mixture was heated in a 70 °C water bath while stirring for ten minutes. The solution was then allowed to cool to room temperature.

Approximately 10 µg of affinity purified rabbit anti-benzoylecgonine antibody (Berkeley Antibody Company, Inc., Richmond, CA) dissolved in PBS (pH 7.4) was added to the selenium suspension (5 ml ). The mixture was gently swirled for about ten minutes. A 5% BSA solution (300 µl) was added to the mixture, and the mixture was gently swirled and then sat for about ten minutes. Five milliliters of the selenium colloid/anti-benzoylecgonine antibody indicator reagent (1.89 µg/ml) was added to five milliliters of conjugate diluent (0.1 M Tris, 4% sucrose, 2% surfactant [Pluronic-68, BASF Corporation, Parsippany, NJ] and 4% casein) for a final concentration of 0.97 µg/ml.

Conjugate diluent was prepared by adding Tris (1.2 g), sucrose (4.0 g) and Pluronic-68 (2.0 g) to distilled water (70 ml) at pH 8.1. Alkaline treated casein (4.0 g) was added to the solution and mixed, and the pH was adjusted to approximately 8.1. Volume was brought to 100 milliliters and filtered through a 0.2 µm filter.

### b) Application pad preparation

Indicator reagent (30 µl, from Example 3.a) was pipetted onto each application pad, and the pads were either air dried at room temperature or lyophilized. The application pad materials were either porous polyethylene (4 mm x 10 mm x 1.5 mm rectangles of Porex material, Porex Technologies, Fairburn, GA) or glass fiber paper (4 mm x 10 mm rectangles of resin bonded glass fiber paper, Lypore-9254, Lydall Incorporated, Rochester, NH).

### c) Chromatographic material preparation

Capture sites were prepared by reagent printing four different reagents as parallel bars, about two millimeters wide and approximately one-quarter inch apart, using sheets of nitrocellulose (8 inch x 10 inch, 5.0 µM, Schleicher and Schuell, Keene, NH). The capture sites were printed substantially in accordance with the method described in Example 1.c using an approximately 50 micron diameter orifice (at 8 psi) and passing the material under the orifice at a rate of four inches per second, with reagent dispensed at about 30 microliters per nine inch bar. The most proximal bar was benzoylecgonine:BSA (17:1) at 1 mg/ml. The next bar was goat anti-rabbit antibody (affinity isolated, Sigma Chemical Company, St. Louis, MO) at 0.5 mg/ml. The next bar was goat anti-mouse antibody (affinity isolated, Sigma) at 0.5 mg/ml. The final bar was a blend of equal portions of both the goat anti-rabbit antibody and the goat anti-mouse antibody. The filter was then cut into 3 mm x 50 mm strips such that the cut was perpendicular to the printed bars. The application pads of Example 3.b were placed in fluid-flow contact with the proximal ends of the chromatographic strips.

### d) Assay protocol

Benzoylecgonine test samples (30 µl of 0 µg/ml or 5.0 µg/ml sample concentrations) were contacted to the application pad. The test sample passed through the application pad wherein any benzoylecgonine in the test sample bound to the reconstituted indicator reagent and migrated through the strip. The indicator reagent/analyte complex bypassed the benzoylecgonine capture site and was immobilized at the goat anti-rabbit antibody bar where the formation of the immunocomplex was detectable by the appearance of a dark signal over substantially the entire bar. The test sample containing no benzoylecgonine reconstituted the indicator reagent, and that unbound indicator reagent was substantially captured at the benzoylecgonine capture site producing a visible sharp thin line. A very faint signal was barely detectable at the goat anti-rabbit antibody bar.

### Example 4

### One Step Immunochromatographic Assay For Opiates

### a. Indicator reagent preparation - selenium colloid/anti-morphine antibody conjugate

A selenium colloid/antibody conjugate was prepared substantially in accordance with the procedure of Example 3.a using a selenium suspension and sheep anti-morphine antibody (100 µg in 0.05M Tris) and a conjugate diluent which included 0.5% polyethylene glycol (0.5 g, MW 20,000, Sigma) for a final concentration of 9.7 µg/ml.

### b) Application pad preparation

Glass fiber paper strips (10.5 cm x 17 mm, Lypore, Lydall Inc.) were soaked with one milliliter of the indicator reagent (from Example 4.a). The strips were suspended in a 45 °C oven for 1 to 1.5 hours and were then cut into 3 mm x 17 mm application pads.

### c) Chromatographic material preparation

The chromatographic material was prepared substantially in accordance with the procedure described in Example 3.c using two different reagents to produce the parallel bars. The first bar was morphine-3-B-D-glucuronide-BSA in PBS (1.206 mg/ml at pH 7.4, Sigma). The second bar was anti-mouse lgG (0.67 mg/ml, Sigma) in Tris (containing 0.1% BSA, 20% glycerol, 0.02% NaN₃ and trace phenol red at pH 8.0).

### d) Assay protocol

Morphine test samples (30 µl of 0, 100, 200, 300, 500 and 1000 ng/ml sample concentrations) were contacted to the application pad. The test sample passed through the application pad wherein any morphine in the test sample bound to the reconstituted indicator reagent and migrated through the strip. The indicator reagent/analyte complex bypassed the morphine-3-B-D-glucuronide-BSA capture site and was immobilized at the goat anti-mouse antibody bar where the formation of the immunocomplex was detectable by the appearance of a dark signal. The test sample containing no morphine reconstituted the indicator reagent which was substantially captured at the morphine-3-B-D-glucuronide-BSA capture site producing a sharp thin line. A very faint signal was barely detectable at the goat anti-mouse antibody bar.

### Example 5

### One Step Immunochromatographic Assay For Hepatitis B Surface Antigen (HBsAg)

### a. Indicator reagent preparation - selenium colloid/anti-biotin antibody conjugate

A selenium colloid suspension was prepared substantially in accordance with the procedure of Example 3.a. Two milliliters of the selenium suspension was mixed with 150 mM borate (44 µl pH 8.0). Rabbit anti-biotin antibody (6 µl, 3 µg/ml) was added, and the mixture was gently mixed for about ten minutes. Ten percent BSA (60 µl) was then added and mixed. The resulting conjugate was used without centrifugation. The selenium colloid/anti-biotin antibody conjugate (0.4 ml) was mixed with 5% casein for assay use.

### b) Application pad preparation

Indicator reagent (30 µl from Example 5.a) was pipetted onto porous polyethylene pads (3/8 inch x 3/16 inch, Porex). The pads were dried at 40 °C for one hour.

### c) Ancillary pad preparation

An ancillary specific binding member complex of biotin and anti-HBsAg antibody was prepared in accordance with procedures well-known in the art. The biotin/anti-HBsAg antibody complex (0.4 ml at 4 µg/ml) was mixed with a diluent (0.4 ml, phosphate buffered saline with 1% BSA). This mixture was also pipetted onto porous polyethylene pads (3/8 inch x 3/16 inch, Porex) which were dried at 40 °C for 1 hour.

### d) Chromatographic material preparation

The chromatographic material was prepared by dotting anti-HBsAg antibody (0.3 µl, 1.83 mg/ml) onto nitrocellulose (Schleicher and Schuell) to form the capture situs. The application pad of Example 5.b was placed upon the proximal end of the chromatographic material, and the ancillary pad of Example 5.c was stacked upon the application pad.

### e) Assay protocol

HBsAg test samples (120 µl of 0, 1.0, 2.5, 5.0, 10.0 and 5000 ng/ml sample concentrations) were contacted to the ancillary pad. The test sample passed through both the ancillary pad and the application pad thereby reconstituting both the biotin-anti-HBsAg antibody complex and the selenium colloid/anti-biotin antibody indicator reagent. If HBsAg was present in the test sample, then a selenium colloid/anti-biotin antibody/biotin-anti-HBsAg antibody/analyte complex was formed and migrated through the strip. The complex was immobilized at the anti-HBsAg antibody capture situs where the formation of a "sandwich" immunocomplex was detectable by the appearance of a dark signal. The 5000 ng/ml test sample resulted in a well-defined dark spot on the chromatographic material. The 5.0 and 10.0 ng/ml test samples resulted in visible spots on the chromatographic material. The 2.5 ng/ml test sample resulted in a very faint spot on the chromatographic material. And, the 0 and 1.0 ng/ml test samples provided no visible color on the chromatographic material.

### Example 6

### One Step Immunochromatographic Assay For hCG

### a. Indicator reagent preparation - selenium colloid/anti-hCG antibody conjugate

A selenium colloid suspension was prepared as a one step procedure substantially in accordance with the procedure of Example 3.a. The selenium suspension (250 ml) was mixed with anti-hCG antibody (1250 µg), and the mixture was gently stirred for about 15 minutes. A BSA blocking solution (25 ml, comprising 5 gm of BSA and 5 ml of 1 M Tris at pH 7.4 with a sufficient quantity of water to bring the volume to 500 ml) was then added to the mixture and stirred. The resulting selenium colloid/anti-hCG antibody conjugate was centrifuged (at 1900 x g) for 40 minutes at 4° C. The supernatant was removed, and BSA blocking solution (25 ml) was added to resuspend the pellet by vortexing. The centrifugation and aspiration process was repeated, and the final pellet was resuspended in BSA blocking solution (5 ml).

### b) Application pad preparation

Indicator reagent (from Example 6.a) was diluted 1:30 with 145 milliliters of conjugate diluent comprising Tris (0.18 g, 10 mM at pH 7.4), 2% lactose (3.0 g) to decrease particle aggregation, alkaline treated casein (2%, 3.0 g) and goat lgG (300 mg, 2.0 mg/ml). The application pad material comprised resin bonded glass fiber paper (Lydall). The indicator reagent was coated onto the application pad material, the material was cut into 1/4 inch x 1/2 inch rectangles, and the pads were dried at 98° F.

### c) Chromatographic material preparation

The capture situs was prepared by reagent printing anti-hCG antibody (0.1 mg/ml in TBS capture antibody diluent containing 0.1M Tris HCl at pH 7.4, 1.0% sucrose, and 0.1% BSA) onto nitrocellulose sheets (8 inch x 10 inch, 5.0 µM Schleicher and Schuell). The sheet was then cut into 6 mm x 48 mm strips such that the cut was perpendicular to the 0.33 mm x 6.0 mm printed bar which contained approximately 0.25 µl of antibody per bar. The application pads of Example 6.b were placed over the bottom 3 mm of the chromatographic strips, approximately 10 mm below the capture situs. The assay device was then enclosed in a holder leaving the top of the application pad exposed.

### d) Assay protocol

Test samples (0, 5.0, 50, 250 and 500 mlU/ml sample concentrations) were contacted to the application pad. The device was either dipped into the test sample to allow the application pad to hydrate or 60 µl of the test sample was added directly to the pad. The test sample passed through the application pad thereby reconstituting the selenium colloid/anti-hCG antibody indicator reagent. If hCG was present in the test sample, then a selenium colloid/anti-hCG antibody/analyte complex was formed and migrated through the strip. The complex was immobilized at the anti-hCG antibody capture situs where the formation of a sandwich immunocomplex was detectable by the appearance of a dark signal. The 500 mlU/ml test sample resulted in a well-defined dark bar on the chromatographic material. The 250 mlU/ml test sample resulted in a lighter bar on the chromatographic material. The 50 mlU/ml test sample resulted in a faint bar on the chromatographic material. And, the 0 and 5.0 mlU/ml test samples provided no visible color on the chromatographic material.

Where technical features mentioned in any claim are followed by reference signs, those reference signs have been included for the sole purpose of increasing the intelligibility of the claims and accordingly, such reference signs do not have any limiting effect on the scope of each element identified by way of example by such reference signs.

## Claims

1. An analytical device (10) for determining the presence or amount of an analyte in a test sample, comprising:
a) an application pad (1) which absorbs the test sample; and
b) a length of chromatographic material (2) in the form of a strip having a proximal end (11) and a distal end (12), wherein said proximal end is in fluid flow contact with said application pad and wherein the test sample in said application pad is induced to travel from said proximal end to said distal end by capillary action, said chromatographic material containing a capture reagent comprising a specific binding member immobilized in a discrete capture situs (7) between said proximal and distal ends,
characterized in that said application pad absorbs a volume of test sample that is equal to or greater than the total volume capacity of the chromatographic material and contains an indicator reagent comprising a label attached to a specific binding member, whereby the test sample and said indicator reagent are mixed by said application pad upon absorption of the test sample and whereby said indicator reagent is transported by the test sample from said application pad and through said capture situs, whereby the binding of said indicator reagent results in a detectable signal at said capture situs thereby indicating the presence or amount of the analyte in the test sample.

2. The device according to Claim 1, wherein said capture reagent is uniformly immobilized within said chromatographic material to form said capture situs.

3. The device according to Claim 1, wherein said capture reagent is immobilized within said chromatographic material to form at least one discrete capture situs substantially smaller in area than said chromatographic material.

4. The device according to Claim 1 , wherein said capture reagent is immobilized within said chromatographic material to form more than one separate parallel capture sites traversing the width of said chromatographic material.

5. The device according to Claim 4, wherein said indicator reagent is a colloid-labeled antibody with a dissociation constant of less than the lowest analyte concentration to be detected.

6. The device according to Claim 1, wherein said capture reagent is immobilized within said chromatographic material in a gradient pattern.

7. The device according to Claim 1, wherein said capture reagent is immobilized within said chromatographic material to form at least one thin stripe.

8. The device according to Claim 1 wherein said capture reagent is attached to microparticles, and wherein said microparticles are immobilized in said chromatographic material.

9. The device according to Claim 1, wherein said capture reagent is a specific binding member of a specific binding pair selected from the group consisting of biotin and avidin, carbohydrates and lectins, complementary nucleotide sequences, complementary peptide sequences, effector and receptor molecules, enzyme cofactors and enzymes, enzyme inhibitors and enzymes, a peptide sequence and an antibody specific for the sequence and immunoreactants.

10. The device according to Claim 1, wherein said label comprises a member selected from the group consisting of chromagens, catalysts, fluorescent compounds, chemiluminescent compounds, radioactive isotopes, colloidal metallic particles, selenium particles, dye particles, enzymes, substrates, organic polymer latex particles and liposomes or other vesicles containing signal producing components.

11. The device according to Claim 1, further comprising at least one signal producing component immobilized with said capture reagent on said chromatographic material.

12. The device according to Claim 1, wherein said application pad comprises a member selected from the group consisting of nitrocellulose, polyethylene frit and glass fiber filter paper.

13. The device according to Claim 1, wherein said chromatographic material comprises a member selected from the group consisting of cellulose, cellulose derivatives, nitrocellulose, fiberglass, cloth, porous gels, porous fibrous matrixes, starch based materials, ceramic materials and films.

14. The device according to Claim 1, wherein said chromatographic material is a nitrocellulose membrane and said application pad is a glass fiber paper.

15. The device according to Claim 1, further comprising a housing enclosing said application pad, thereby preventing the test sample from directly contacting said chromatographic material, wherein said housing has a means for applying test sample to said application pad.

16. The device according to Claim 1, further comprising at least one ancillary specific binding member, wherein at least one of said capture reagent and said indicator reagent is specific for said ancillary specific binding member.

17. An analytical method for determining the presence or amount of an analyte in a test sample, using a chromatographic device (10) according to Claim 1, comprising the steps of:
a. contacting said application pad (1) with the test sample suspected of containing the analyte, wherein the test sample contacts said indicator reagent, and wherein the test sample and said indicator reagent are transferred to said chromatographic material (2);
b. allowing the test sample and said indicator reagent to migrate through said chromatographic material and contact said capture reagent resulting in the production of a detectable signal at said capture situs (7); and
c. observing the detectable signal to determine the presence or amount of an analyte in the test sample.

18. The method according to Claim 17, further comprising, simultaneously or sequentially adding a wicking solution with the test sample to said application pad.

19. The method according to Claim 17, wherein said capture reagent is immobilized within said chromatographic material to form at least one discrete capture situs substantially smaller in area than said chromatographic material.

20. The method according to Claim 17, wherein said capture reagent is immobilized within said chromatographic material to form more than one separate parallel capture sites traversing the width of said chromatographic material.

21. The method according to Claim 17, wherein said capture reagent is immobilized within said chromatographic material in a gradient pattern.

22. A test kit, containing the assay device of any of preceding Claims 1 to 16.

23. A test kit according to Claim 22, further containing a wicking solution.

24. A test kit according to Claim 22, further containing a test sample pretreatment reagent.

## Patentansprüche

1. Analytische Vorrichtung (10) zur Bestimmung des Vorliegens oder der Menge eines Analyten in einer Testprobe, umfassend:
(a) ein Auftragekissen (1), das die Testprobe absorbiert; und
(b) eine Länge chromatographisches Material (2) in Form eines Streifens mit einem nahen Ende (11) und einem fernen Ende (12), worin das genannte nahe Ende in Flüssigkeits-Fließkontakt mit dem genannten Auftragekissen steht und worin die Testprobe in dem genannten Auftragekissen durch Kapillarwirkung veranlaßt wird, von dem genannten nahen Ende zu dem genannten fernen Ende zu wandern, wobei das genannte chromatographische Material ein Fangreagens enthält, welches ein spezifisches Bindeglied umfaßt, das an einer diskreten Fangstelle (7) zwischen dem genannten nahen und fernen Ende immobilisiert ist,
dadurch gekennzeichnet, daß das genannte Auftragskissen ein Volumen der Testprobe absorbiert, das gleich oder größer ist als die gesamte Volumenkapazität des chromatographischen Materials, und ein Indikatorreagens enthält, welches eine Markierung umfaßt, die an ein spezifisches Bindeglied angeknüpft ist, wobei die Testprobe und das genannte Indikatorreagens durch das genannte Auftragekissen bei Absorption der Testprobe gemischt werden und wobei das genannte Indikatorreagens von der Testprobe von dem genannten Auftragekissen und durch die genannte Fangstelle transportiert wird, wobei das Binden des genannten Indikatorreagens zu einem nachweisbaren Signal an der genannten Fangstelle führt, wodurch das Vorliegen oder die Menge des Analyten in der Testprobe angezeigt wird.

2. Vorrichtung nach Anspruch 1, worin das genannte Fangreagens einheitlich in dem genannten chromatographischen Material unter Bildung der genannten Fangstelle immobilisiert ist.

3. Vorrichtung nach Anspruch 1, worin das genannte Fangreagens in dem genannten chromatographischen Material immobilisiert ist unter Bildung von mindestens einer diskreten Fangstelle, die in der Fläche im wesentlichen kleiner ist als das genannte chromatographische Material.

4. Vorrichtung nach Anspruch 1, worin das genannte Fangreagens in dem genannten chromatographischen Material immobilisiert ist unter Bildung von mehr als einer getrennten parallelen Fangstelle, die über die Breite des chromatographischen Materials verläuft.

5. Vorrichtung nach Anspruch 4, worin das genannten Indikatorreagens ein kolloidmarkierter Antikörper mit einer Dissoziationskonstante von weniger als der niedrigsten nachzuweisenden Analytenkonzentration ist.

6. Vorrichtung nach Anspruch 1, worin das genannte Fangreagens in dem genannten chromatographischen Material in einem Form eines Gradientprofils immobilisiert ist.

7. Vorrichtung nach Anspruch 1, worin das Fangreagens in dem genannten chromatographischen Material unter Bildung von mindestens einem dünnen Streifen immobilisiert ist.

8. Vorrichtung nach Anspruch 1, worin das genannte Fangreagens an Mikroteilchen gebunden ist und worin die genannten Mikroteilchen in dem genannten chromatographischen Material immobilisiert sind.

9. Vorrichtung nach Anspruch 1, worin das genannte Fangreagens ein spezifisches Bindeglied eines spezifischen Bindungspaares darstellt, ausgewählt aus der Gruppe, bestehend aus Biotin und Avidin, Kohlehydraten und Lectinen, komplementären Nucleotidsequenzen, komplementären Peptidsequenzen, Effektor- und Rezeptormolekülen, Enzymcofaktoren und Enzymen, Enzymhemmstoffen und Enzymen, einer Peptidsequenz und einem für die Sequenz spezifischen Antikörper und Immunreagentien.

10. Vorrichtung nach Anspruch 1, worin die genannte Markierung ein Glied umfaßt, ausgewählt aus der Gruppe bestehend aus Chromagenen, Katalysatoren, fluoreszierenden Verbindungen, chemoluminiscenten Verbindungen, radioaktiven Isotopen, kolloidalen metallischen Teilchen, Selenteilchen, Farbstoff-Teilchen, Enzymen, Substraten, organischen polymeren Latexteilchen und Liposomen oder anderen Vesikeln, die Signal-produzierende Komponenten enthalten.

11. Vorrichtung nach Anspruch 1, die ferner mindestens eine Signal-produzierende Komponente umfaßt, die mit dem genannten Fangreagens auf dem genannten chromatographischen Material immobilisiert ist.

12. Vorrichtung nach Anspruch 1, worin das genannte Auftragekissen ein Glied umfaßt, ausgewählt aus der Gruppe bestehend aus Nitrocellulose, Polyethylenfritte und Glasfaser-Filterpapier.

13. Vorrichtung nach Anspruch 1, worin das genannte chromatographische Material ein Glied umfaßt, ausgewählt aus der Gruppe bestehend aus Cellulose, Cellulosederivaten, Nitrocellulose, Fiberglas, Stoffgewebe, porösen Gelen, porösen Faser-Matrices, Materialien aus Stärkebasis, keramischen Materialien und Folie.

14. Vorrichtung nach Anspruch 1, worin das genannte chromatographische Material eine Nitrocellulose-Membran darstellt und das genannte Auftragekissen Glasfaserpapier ist.

15. Vorrichtung nach Anspruch 1, die weiterhin umfaßt ein Gehäuse, das das genannte Auftragekissen umschließt, wodurch verhindert wird, daß die Testprobe direkt mit dem genannten chromatographischen Material in Kontakt tritt.

16. Vorrichtung nach Anspruch 1, die weiterhin umfaßt mindestens ein zusätzliches spezifisches Bindeglied, worin mindestens eines von dem genannten Fangreagens und dem genannten Indikator für das genannte zusätzliche spezifische Bindeglied spezifisch ist.

17. Analytisches Verfahren zur Bestimmung des Vorliegens oder der Menge eines Analyten in einer Testprobe unter Verwendung der chromatographischen Vorrichtung (10) nach Anspruch 1, umfassend die Stufen:
(a) Kontaktieren des genannten Auftragekissens (1) mit der Testprobe, von der erwartet wird, daß sie den Analyten enthält, worin die Testprobe mit dem genannten Indikatorreagens in Kontakt tritt und worin die Testprobe und das genannte Indikatorreagens zu dem genannten chromatographischen Material (2) übergeführt werden;
(b) Ermöglichen, daß die Testprobe und das genannte Indikatorreagens durch das genannte chromatographische Material wandern und mit dem genannten Fangreagens in Kontakt treten, was zu einer Produktion eines nachweisbaren Signals an der genannten Fangstelle (7) führt; und
(c) Beobachten des nachweisbaren Signals zur Bestimmung des Vorliegens oder der Menge eines Analyten in der Testprobe.

18. Verfahren nach Anspruch 17, das weiterhin umfaßt Aufgeben einer Dochtlösung auf das genannte Auftragekissen gleichzeitig oder nacheinander mit der Testprobe.

19. Verfahren nach Anspruch 17, bei dem das genannte Fangreagens in dem genannten chromatographischen Material immobilisiert wird unter Bildung von mindestens einer diskreten Fangstelle, die in der Fläche im wesentlichen kleiner ist als das genannte chromatographische Material.

20. Verfahren nach Anspruch 17, bei dem das genannte Fangreagens in dem genannten chromatographischen Material immobilisiert ist unter Bildung von mehr als einer getrennten parallelen Fangstelle, die über die Breite des chromatographischen Materials verläuft.

21. Verfahren nach Anspruch 17, bei dem das genannte Fangreagens in dem genannten chromatographischen Material in Form eines Gradientenprofils immobilisiert wird.

22. Testkit, enthaltend eine Testvorrichtung nach einem der vorgenannten Ansprüche 1 bis 16.

23. Testkit nach Anspruch 22, das ferner eine Dochtlösung enthält.

24. Testkit nach Anspruch 22, das ferner eine Probe als Vorbehandlungsreagens enthält.

## Revendications

1. Dispositif analytique (10) pour déterminer la présence ou la quantité d'un analyte dans un échantillon à tester, comprenant :
a) un tampon d'application (1) qui absorbe l'échantillon à tester, et
b) une longueur de matériau chromatographique (2) sous forme d'une bande ayant une extrémité proximale (11) et une extrémité distale (12), ladite extrémité proximale étant en contact par écoulement de fluide avec ledit tampon d'application et l'échantillon à tester dans ledit tampon d'application étant amené à se déplacer de ladite extrémité proximale à ladite extrémité distale par action capillaire, ledit matériau chromatographique contenant un réactif de capture comprenant un élément de liaison spécifique immobilisé dans un site de capture discret (7) entre lesdites extrémités proximale et distale,
caractérisé en ce que ledit tampon d'application absorbe un volume d'échantillon à tester qui est supérieur ou égal à la capacité volumique totale du matériau chromatographique et contient un réactif indicateur comprenant un traceur fixé à un élément de liaison spécifique, de sorte que l'échantillon à tester et ledit réactif indicateur sont mélangés par ledit tampon d'application lors de l'absorption de l'échantillon à tester et ledit réactif indicateur est transporté par l'échantillon à tester depuis ledit tampon d'application et sur ledit site de capture, de sorte que la liaison dudit réactif indicateur produit un signal détectable audit site de capture qui indique la présence ou la quantité de l'analyte dans l'échantillon à tester.

2. Dispositif selon la revendication 1, dans lequel ledit réactif de capture est immobilisé uniformément dans ledit matériau chromatographique pour former ledit site de capture.

3. Dispositif selon la revendication 1, dans lequel ledit réactif de capture est immobilisé dans ledit matériau chromatographique pour former au moins un site de capture discret de surface sensiblement inférieure à celle dudit matériau chromatographique.

4. Dispositif selon la revendication 1, dans lequel ledit réactif de capture est immobilisé dans ledit matériau chromatographique pour former plusieurs sites de capture parallèles séparés qui s'étendent sur la largeur dudit matériau chromatographique.

5. Dispositif selon la revendication 4, dans lequel ledit réactif indicateur est un anticorps marqué par un colloïde qui a une constante de dissociation inférieure à la plus basse concentration d'analyte à détecter.

6. Dispositif selon la revendication 1, dans lequel ledit réactif de capture est immobilisé dans ledit matériau chromatographique dans une configuration de gradient.

7. Dispositif selon la revendication 1, dans lequel ledit réactif de capture est immobilisé dans ledit matériau chromatographique pour former au moins une bande mince.

8. Dispositif selon la revendication 1, dans lequel ledit réactif de capture est fixé à des microparticules et dans lequel lesdites microparticules sont immobilisées dans ledit matériau chromatographique.

9. Dispositif selon la revendication 1, dans lequel ledit réactif de capture est un élément de liaison spécifique d'une paire de liaison spécifique choisie dans le groupe formé par la biotine et l'avidine, les glucides et les lectines, les séquences nucléotidiques complémentaires, les séquences peptidiques complémentaires, les molécules effectrices et réceptrices, les cofacteurs d'enzymes et les enzymes, les inhibiteurs d'enzymes et les enzymes, une séquence peptidique et un anticorps spécifique de la séquence et les corps immunoréactifs.

10. Dispositif selon la revendication 1, dans lequel ledit traceur comprend un élément choisi dans le groupe formé par les chromogènes, les catalyseurs, les composés fluorescents, les composés chimiluminescents, les isotopes radioactifs, les particules métalliques colloïdales, les particules de sélénium, les particules de colorant, les enzymes, les substrats, les particules de latex polymériques organiques et les liposomes ou d'autres vésicules contenant des constituants producteurs de signal.

11. Dispositif selon la revendication 1, comprenant en outre au moins un constituant producteur de signal immobilisé avec ledit réactif de capture sur ledit matériau chromatographique.

12. Dispositif selon la revendication 1, dans lequel ledit tampon d'application comprend un élément choisi dans le groupe formé par la nitrocellulose, une fritte de polyéthylène et un papier filtre à fibres de verte.

13. Dispositif selon la revendication 1, dans lequel ledit matériau chromatographique comprend un élément choisi dans le groupe formé par la cellulose, les dérivés cellulosiques, la nitrocellulose, les fibres de verte, les tissus, les gels poreux, les matrices fibreuses poreuses, les matériaux à base d'amidon, les matériaux céramiques et les films.

14. Dispositif selon la revendication 1, dans lequel ledit matériau chromatographique est une membrane de nitrocellulose et ledit tampon d'application est un papier à fibres de verte.

15. Dispositif selon la revendication 1, comprenant en outre un boîtier renfermant ledit tampon d'application, pour empêcher l'échantillon à tester d'entrer directement en contact avec ledit matériau chromatographique, ledit boîtier ayant un dispositif pour appliquer l'échantillon à tester sur ledit tampon d'application.

16. Dispositif selon la revendication 1, comprenant en outre au moins un élément de liaison spécifique auxiliaire, au moins un réactif parmi ledit réactif de capture et ledit réactif indicateur étant spécifique dudit élément de liaison spécifique auxiliaire.

17. Procédé analytique pour déterminer la présence ou la quantité d'un analyte dans un échantillon à tester, au moyen d'un dispositif chromatographique (10) selon la revendication 1, comprenant les étapes suivantes :
a. mettre en contact ledit tampon d'application (1) avec l'échantillon à tester supposé contenir l'analyte, l'échantillon à tester entrant en contact avec ledit réactif indicateur et l'échantillon à tester et ledit réactif indicateur étant transférés audit matériau chromatographique (2);
b. amener l'échantillon à tester et ledit réactif indicateur à migrer dans ledit matériau chromatographique et à entrer en contact avec ledit réactif de capture pour produire un signal détectable au niveau dudit site de capture (7) ; et
c. observer le signal détectable pour déterminer la présence ou la quantité d'un analyte dans l'échantillon à tester.

18. Procédé selon la revendication 17, comprenant en outre, simultanément ou successivement, l'addition audit tampon d'application d'une solution à effet de mèche avec l'échantillon à tester.

19. Procédé selon la revendication 17, dans lequel ledit réactif de capture est immobilisé dans ledit matériau chromatographique pour former au moins un site de capture discret de surface sensiblement inférieure à celle dudit matériau chromatographique.

20. Procédé selon la revendication 17, dans lequel ledit réactif de capture est immobilisé dans ledit matériau chromatographique pour former plusieurs sites de capture parallèles séparés qui s'étendent sur la largeur dudit matériau chromatographique.

21. Procédé selon la revendication 17, dans lequel ledit réactif de capture est immobilisé dans ledit matériau chromatographique dans une configuration de gradient.

22. Trousse de test contenant le dispositif de test selon l'une quelconque des revendications 1 à 16 précédentes.

23. Trousse de test selon la revendication 22, contenant en outre une solution à effet de mèche.

24. Trousse de test selon la revendication 22, contenant en outre un réactif de prétraitement de l'échantillon à tester.
